# EUROPÄISCHE PATENTSCHRIFT

(11) **EP 0 766 550 B1**
(45) Veröffentlichungstag und Bekanntmachung des Hinweises auf die Patenterteilung: **11.08.1999**
(21) Anmeldenummer: 95925800.5
(22) Anmeldetag: 30.06.1995
(51) Int. Cl.: A61F 13/20

(54) **TAMPON**
TAMPON
TAMPON

(30) Priorität: 30.06.1994 DE 9410595 U
(43) Veröffentlichungstag der Anmeldung: 09.04.1997
(73) Patentinhaber: Kimberly-Clark GmbH, 56070 Koblenz (DE)
(72) Erfinder: WEINSTRAUCH, Fritz, D-41564 Kaarst (DE)
(74) Vertreter: Diehl, Hermann, Dr. Dipl.-Phys.
(86) Internationale Anmeldenummer: EP9502551
(87) Internationale Veröffentlichungsnummer: WO9600552

(56) Entgegenhaltungen:
- CH-A- 565 553
- US-A- 4 185 631
- US-A- 4 351 339

## Beschreibung

Die Erfindung betrifft einen Tampon, insbesondere für die Frauenhygiene.

Derartige Tampons sind in vielerlei Ausführungsformen seit langem üblich. Als Beispiel für Tampons sind Wickeltampons, sogenannte "Teebeutel-Tampons" oder aus einem Vliesbandabschnitt gefertigte Tampons zu nennen.

US-A-4,351,339 zeigt einen Tampon mit einem absorbierenden Kern, der spiralförmig gewunden ist, und mit einer durchlässigen Hülle, die Falten aufweist. Die Hülle hat eine hohe Permeabilitat, damit Körperflüssigkeit zu dem saugfähigen Kern gelangen kann. Die Falten sind derart übereinandergelegt, daß sich die Falten beim Herausziehen des Tampons glätten, so daß die Hülle wesentlich langer wird. Der spiralförmige Kern wird beim Herausziehen in die Länge gezogen und sein Durchmesser wird dadurch verringert.

US-A-4,185,631 offenbart einen Tampon mit einem zylindrischen absorbierenden Tamponkörper und einem äußeren Ring, der verschiebbar an dem Tamponkörper angebracht ist. Der absorbierende Kern ist in einem Sack aus flüssigkeitsdurchlässigem Material untergebracht. Der Ring umfaßt ein Material, das expandieren wird, wenn er in der Vagina plaziert ist, um ein Vorbeifließen von Menstruationsflüssigkeit zu verhindern.

Ein grundsätzliches Problem, das bei praktisch allen Tampon-Varianten trotz deren umfassender Weiterentwicklung im Hinblick auf die Auslaufsicherheit und das Flüssigkeitsaufnahmevermögen auftritt, ist die sogenannte "frühe Leckage". Darunter ist ein Versagen des Tampons in der Zeitspanne kurz nach dem Einführen zu verstehen. In diesem Zustand ist der Tampon nämlich noch nicht durch

Flüssigkeitsaufnahme aufgequollen, so daß er seine in diesem Zustand zum Tragen kommende Sperrfunktion für Menstruationsflüssigkeiten im Vaginalkanal noch nicht erfüllen kann. Seitlich des Tampons verbleiben nämlich noch Freiräume, durch die Menstruationsflüssigkeit durchtreten kann.

Ausgehend von der geschilderten Problematik liegt der Erfindung die Aufgabe zugrunde, einen Tampon so auszugestalten, daß seine Sicherheit gegen ein Versagen allgemein und insbesondere kurz nach dem Einsetzen des Tampons in den Vaginalkanal erhöht wird.

Diese Aufgabe wird durch den Tampon gemäß dem unabhängigen Anspruch 1 gelöst. Weitere vorteilhafte Ausgestaltungen, Merkmale, Aspekte oder Details der Erfindung ergeben sich aus den abhängigen Ansprüchen, der Beschreibung und den Zeichnungen. Der erfindungsgemäße Tampon weist Barriere-Streifen auf, die sich mit dem Einsetzen des Tampons im Vaginalkanal von der Tamponoberfläche abspreizen und damit die zu diesem Zeitpunkt noch vorhandenen Freiräume zwischen der Tamponoberfläche und der Vaginalwand zumindest zu einem großen Teil verschließen.

Grundsätzlich können diese flexiblen Barriere-Streifen in Längsrichtung des Tampons an dessen Oberfläche oder in einer spiralförmigen Konfiguration verlaufen. Besonders bevorzugt ist jedoch, wenn, beispielsweise bei einem rotationssymmetrisch ausgebildeten Wickel- oder Beuteltampon, die Barriere-Streifen in Umfangsrichtung um den Tampon verlaufen und radial abspreizbar sind. Derartige Barriere-Streifen sind besonders wirkungsvoll, da sie die oben erwähnten Freiräume zwischen Tamponoberfläche und Vaginalwand zuverlässig verschließen können. Darüber hinaus können solche in Umfangsrichtung verlaufenden Barriere-Streifen herstellungstechnisch besonders einfach auf der Tamponoberfläche angebracht werden, was im Hinblick auf die Tatsache besonders vorteilhaft ist, daß es sich bei Tampons um ausgesprochene Massenartikel handelt.

Eine besonders rationell zu fertigende Variante der Barriere-Streifen erhält man, wenn diese Streifen durch Ausfaltungen des Hüllenmaterials selbst gebildet sind. Damit braucht in der Fertigungsmaschine für beispielsweise einen Wickeltampon lediglich ein etwas breiterer Hüllvliesabschnitt über einen entsprechenden Faltenleger zugeführt zu werden, um einen erfindungsgemäßen Tampon zu realisieren.

Bevorzugt können die Barriere-Streifen natürlich auch aus gesonderten Streifen gebildet sein, die durch Verkleben, Heißsiegeln, Vernadeln, Aufprägen oder dergleichen auf die Hülle aufgebracht sind.

Die Barriere-Streifen beim erfindungsgemäßen Tampon können ihre Sicherungsfunktion auf unterschiedliche Weise erfüllen.

So können die Barriere-Streifen im aufgespreizten Zustand hauptsächlich als mechanische Sperren wirken und die eingangs erwähnten Freiräume seitlich des Tampons blockieren. In diesem Zusammenhang ist es von Vorteil, wenn die Barriere-Streifen aus einem hydrophoben Vliesstoffmaterial bestehen, da durch die damit verbundene flüssigkeitsabweisende Wirkung ein zusätzlicher Sperreffekt erzielt wird.

Weiterhin können die Barriere-Streifen als zusätzliche Speicher für Körperflüssigkeiten bzw. als Leiteinrichtungen für die Körperflüssigkeiten zur Tamponoberfläche hin fungieren. In diesem Zusammenhang ist eine hydrophile Ausrüstung des die Barriere-Streifen bildenden Vliesstoffmaterials von Vorteil.

Die vorstehend erwähnte Saugwirkung der Barriere-Streifen wird durch eine Füllung aus saugfähigem Material noch verstärkt. Als saugfähiges Material können beispielsweise ein Watteband, Zellstoff-Flocken oder dergleichen verwendet werden, die gegebenenfalls noch mit einem sogenannten "Superabsorber" versetzt sein können.

Durch die im Anspruch 8 angegebene dauerhafte oder lösbare Fixierung der freien Randkanten der Barriere-Streifen an der Tamponhülle kann einerseits das Aufspreizverhalten der Barriere-Streifen gesteuert werden. Darüber hinaus können durch diese Fixierungen durch die Barriere-Streifen Taschen an der Außenseite des Tampons ausgebildet werden, in denen z.B. feste Anteile der Menstruationsflüssigkeit besonders wirkungsvoll zurückgehalten werden können.

Weitere Merkmale, Einzelheiten und Vorteile der Erfindung sind der nachfolgenden Beschreibung entnehmbar, in der Ausführungsbeispiele des Erfindungsgegenstands anhand der beigefügten Zeichnungen näher erläutert werden. Es zeigen:
- Fig. 1: eine perspektivische Darstellung eines Wickeltampons,
- Fig. 2: eine schematische Darstellung des Tampons nach Fig. 1 im Anwendungsfall,
- Fig. 3 bis 5: perspektivische Darstellungen von Herstellungszwischenschritten bei der Fertigung des Tampons nach Fig. 1,
- Fig. 6 und 7: erfindungsgemäße Tampons in zwei weiteren Ausführungsformen, und
- Fig. 8: eine perspektivische Darstellung des Tampons nach Fig. 7 in einem Fertigungszwischenschritt.

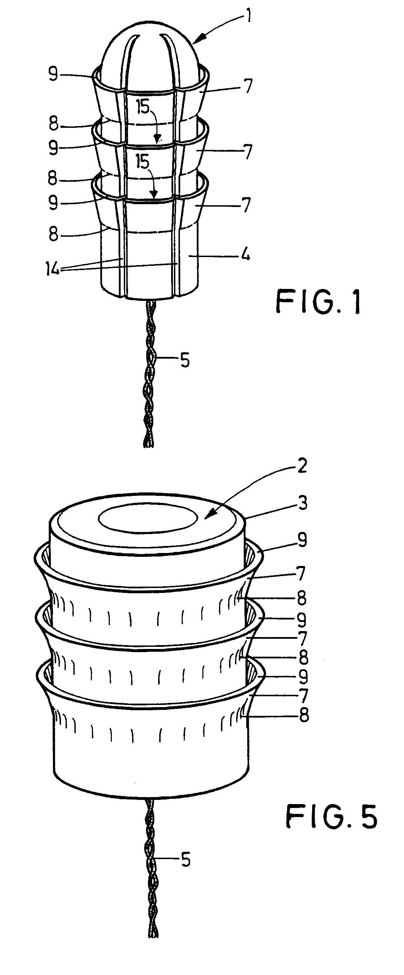

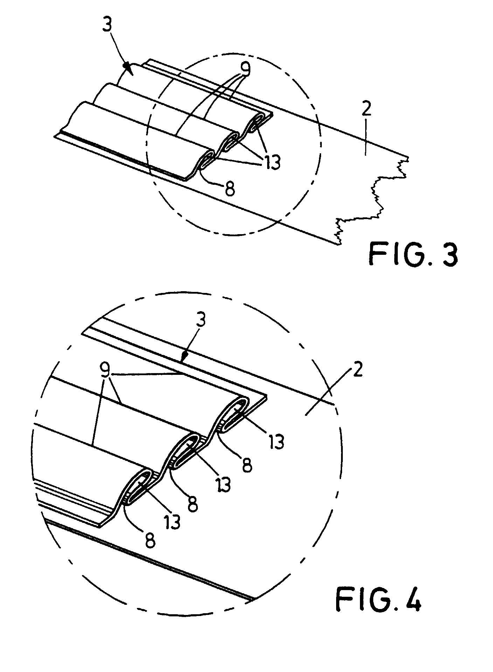

Der in Fig. 1 gezeigte Wickeltampon 1 entspricht in seiner Grundkonstruktion völlig den üblichen Wickeltampons, d.h., daß der Tampon 1 einen saugfähigen Kern aus einem auf sich selbst gewickelten Watteband 2 (Fig. 3 bis 5) aufweist, um den eine aus einem Vliesstoff-Streifen 3 (Fig. 3 bis 5) gebildete Hülle 4 angeordnet ist. Des weiteren ist ein mit dem Kern verbundener Rückholfaden 5 an dem der Rundkuppe 6 gegenüberliegenden Ende des Wickeltampons 1 vorgesehen.

An der Hülle 4 sind über deren Länge verteilt drei in Umfangsrichtung des Wickeltampons 1 umlaufende Barriere-Streifen 7 vorgesehen, deren entgegen der Einführungsrichtung E des Wickeltampons 1 weisender, in Umfangsrichtung verlaufender Rand 8 in noch näher zu erläuternder Weise einstückig in die Hülle 4 übergeht. Bis zu dem gegenüberliegenden, in Einführungsrichtung E weisenden Rand 9 sind die einzelnen Barriere-Streifen 7 lose, so daß sich die Barriere-Streifen 7 von der Tamponoberfläche abspreizen können.

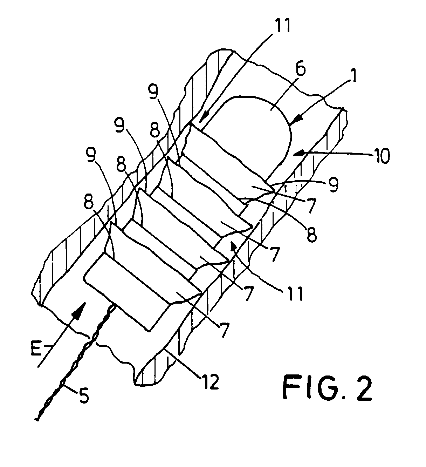

Dies ist in Fig. 2 schematisch dargestellt. Wie daraus deutlich wird, verschließen die sich beim Einführen des Wickeltampons 1 in den Vaginalkanal 10 radial nach außen abspreizenden Barriere-Streifen 7 den Freiraum 11 zwischen der Außenseite des Wickeltampons 1 und der Vaginalwand 12. Die Barriere-Streifen 7 bilden also einerseits eine mechanische Sperre gegen ein Durchtreten von Menstruationsflüssigkeit durch diesen Freiraum 11. Darüber hinaus saugen die Barriere-Streifen 7 selbst Menstruationsflüssigkeit auf, da sie beispielsweise aus einem hydrophilen, saugfähigen Material gefertigt sein können. Nicht zuletzt leiten die Barriere-Streifen 7 die Menstruationsflüssigkeit in Richtung zur Tamponoberfläche, so daß dieser schneller Flüssigkeit aufnehmen und damit aufquellen kann.

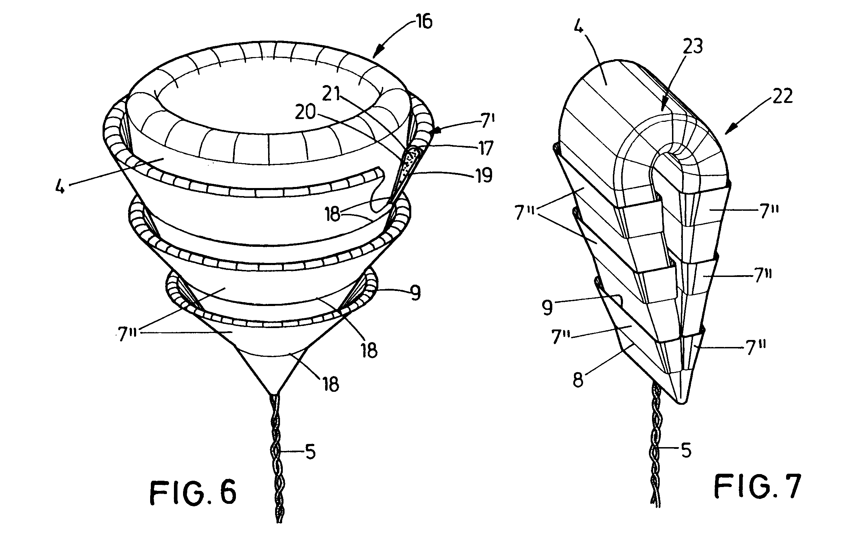

Wie aus den Fig. 3 und 5 deutlich wird, sind die bei dem in Fig. 1 gezeigten Wickeltampon 1 vorhandenen Barriere-Streifen 7 durch Ausfaltungen des Hüllmaterials gebildet. Es wird nämlich der Vliesstoff-Streifen 3, der die Hülle 4 des Tampons bildet, bei der Fertigung in Ausfaltungen oder Falten 13 auf dem den Kern bildenden Watteband 2 befestigt. Die Faltenrichtung verläuft dabei in Längsrichtung des Wattebandes 2 bzw. Vliesstoff-Streifens 3, so daß sich nach dem Aufwickeln des Wattebandes 2 und Vliesstoff-Streifens 3 die Barriere-Streifen 7 in Umfangsrichtung um den Tampon erstrecken (Fig. 5).

Nach dem Aufwickeln des Wattebandes 2 und des in Falten 13 gelegten Vliesstoff-Streifens 3 wird letzterer in üblicher Weise, z.B. durch Heißprägen, auf sich selbst fixiert, so daß der in Fig. 5 gezeigte Tampon-Rohling entsteht. Dieser wird ebenfalls in üblicher Weise in einem Preßwerkzeug auf die in Fig. 1 gezeigte Form verpreßt, wobei durch eine entsprechende Ausbildung des Preßwerkzeuges in Längsrichtung des Wickeltampons 1 verlaufende beabstandete Bereiche oder Rillen 14 mit eingeprägt werden. Durch diese über den Umfang des Wickeltampons 1 verteilten Rillen 14 werden ferner die freien Ränder 9 der Barriere-Streifen 7 im Bereich der Rillenprägung an der Tamponhülle 4 fixiert, wobei dies je nach Stärke der Rillenprägung dauerhaft oder nur vorübergehend sein kann. Durch diese Fixierung bilden sich zwischen den Rillen 14 Taschen 15 im Bereich der Barriere-Streifen 7 aus.

In Fig. 6 ist ein sogenannter Beuteltampon 16 dargestellt, dessen Grundkonstruktion ebenfalls üblich ist und daher keiner näheren Erläuterung bedarf. An der mit Saugmaterial (z.B. Zellstoff-Flocken) gefüllten Hülle 4 des kegelförmigen Tamponkörpers sind wiederum Barriere-Streifen 7' angeordnet, die in Umfangsrichtung um den Tamponkörper herum verlaufen. Im Gegensatz zu dem Wickeltampon nach Fig. 1 sind hier die Barriere-Streifen 7' durch von der Hülle 4 gesonderte Vliesstoff-Streifen 17 gebildet, die bei der Herstellung auf sich selbst gefaltet und im Bereich ihrer entgegen der Einführungsrichtung E weisenden freien Ränder 18 gemeinsam und mit der Hülle 4 verklebt sind. Der Raum zwischen den beiden Lagen 19, 20 des Barriere-Streifens 7' ist dabei mit einem saugfähigen Material (z.B. Zellstoff-Flocken 21) gefüllt. Die Vliesstoff-Streifen 17 selbst können z.B. aus einem hydrophil ausgerüsteten Polypropylen-spunbond-Vliesstoff bestehen. Es sind jedoch auch sämtliche anderen, bei der Tamponfertigung üblichen Vliesstoff-Materialien einsetzbar.

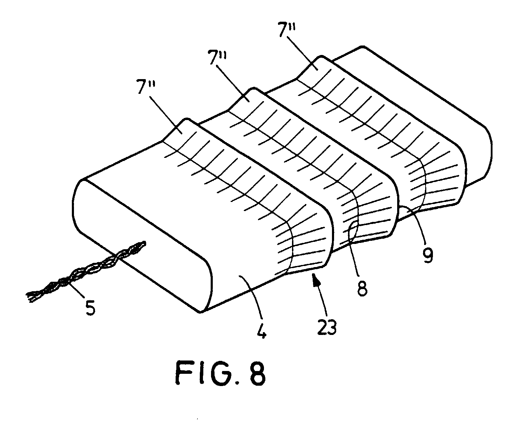

In Fig. 7 ist als weitere Ausführungsform der Erfindung ein Tampon 22 gezeigt, der aus einem U-förmig auf sich selbst gefalteten, quaderförmigen Tamponkörper 23 besteht. Letzterer ist in üblicher Weise aus einem saugfähigen Kern (nicht dargestellt) und einer Hülle 4 gefertigt. Der Kern wird dabei aus einem zu einem Lagenpaket gelegten Vliesband gebildet. Die Hülle weist wiederum durch entsprechende Faltenlegung einstückig damit verbundene Barriere-Streifen 7'' auf, die um den in Fig. 8 teilweise dargestellten Tamponrohling herum verlaufen.

Generell ist darauf hinzuweisen, daß die Höhe h (Fig. 1) der Barriere-Streifen 7, 7', 7'' etwa 5 bis maximal 25 mm betragen kann. Die Anzahl der Barriere-Streifen 7, 7', 7'' kann von 1 bis vorzugsweise etwa 5 variieren.

## Patentansprüche

1. Tampon, insbesondere für die Frauenhygiene, mit
einem saugfähigen Kern,
einem mit dem Kern verbundenen Rückholfaden (5), der entgegen der Einführungsrichtung weist,
einer für Körperflüssigkeiten durchlässigen Hülle (4) um den Kern,
wobei die Hülle (4) mit von der Tamponoberfläche abspreizbaren, bevorzugt flexiblen Barriere-Streifen (7; 7'; 7'') versehen ist, die jeweils einen freien Rand (9) aufweisen,
**dadurch gekennzeichnet**, daß
die freien Ränder (9) der Barriere-Streifen (7; 7'; 7'') in Einführungsrichtung weisen, so daß sich die Barriere-Streifen (7; 7'; 7'') beim Einführen in den Vaginalkanal von der Tamponoberfläche abspreizen und einen Freiraum (11) zwischen der Außenseite des Tampons und der Vaginalwand verschließen.

2. Tampon nach Anspruch 1, dadurch gekennzeichnet, daß die Barriere-Streifen (7,7') in Umfangsrichtung um den Tampon (1,16) verlaufen und radial abspreizbar sind.

3. Tampon nach Anspruch 1 oder 2, dadurch gekennzeichnet, daß die Barriere-Streifen (7,7'') durch Ausfaltungen (13) des Hüllenmaterials (3) gebildet sind.

4. Tampon nach Anspruch 1 oder 2, dadurch gekennzeichnet, daß die Barriere-Streifen (7') auf die Hülle (4) durch Verkleben, Heißsiegeln, Vernadeln, Aufprägen oder dergleichen aufgebracht sind.

5. Tampon nach einem der Ansprüche 1 bis 4, dadurch gekennzeichnet, daß die Barriere-Streifen (7,7',7'') aus hydrophobem Vliesstoffmaterial bestehen.

6. Tampon nach einem der Ansprüche 1 bis 4, dadurch gekennzeichnet, daß die Barriere-Streifen (7,7',7'') aus hydrophilem Vliesstoffmaterial bestehen.

7. Tampon nach einem der Ansprüche 1 bis 6, dadurch gekennzeichnet, daß die Barriere-Streifen (7') zweilagig ausgebildet sind und eine Füllung (Zellstoff-Flocken 21) aus saugfähigem Material aufweisen.

8. Tampon nach einem der Ansprüche 1 bis 7, dadurch gekennzeichnet, daß die freien Ränder (9) der Barriere-Streifen (7) in voneinander beabstandeten Bereichen (14) an der Tamponhülle (4) dauerhaft oder lösbar fixiert sind.

## Claims

1. A tampon, especially for feminine care, comprising
an absorbent core,
a withdrawal cord (5) which is connected to said core and oriented opposite to the direction of insertion,
a cover (4) which is disposed around said core and is permeable to body fluids,
said cover (4) comprising preferably flexible barrier strips (7;7';7'') which are spreadable from the tampon surface, each of said barrier strips exhibiting a free edge (9),
**characterized**
in that said free edges (9) of said barrier strips (7;7',7'') are oriented in the direction of insertion so that, when the tampon is inserted into the vaginal duct, said barrier strips (7;7';7'') spread from the tampon surface and close a free space (11) between the outside of the tampon and the vaginal wall.

2. Tampon according to claim 1, characterized in that the barrier strips (7, 7') extend around the tampon (1, 16) in circumferential direction and are radially spreadable.

3. Tampon according to claim 1 or 2, characterized in that the barrier strips (7, 7'') are formed from foldings (13) of the cover material (3).

4. Tampon according to claim 1 or 2, characterized in that the barrier strips (7') are applied to said cover (4) by means of gluing, heat sealing, needle punching, embossing or the like.

5. Tampon according to one of claims 1 to 4, characterized in that the barrier strips (7, 7', 7'') consist of a hydrophobic nonwoven material.

6. Tampon according to one of claims 1 to 4, characterized in that the barrier strips (7, 7', 7'') consist of a hydrophilic nonwoven material.

7. Tampon according to one of claims 1 to 6, characterized in that the barrier strips (7') are of two-layered construction and comprise a filling (wood pulp fluff 21) of absorbent material.

8. Tampon according to one of claims 1 to 7, characterized in that the free edges (9) of the barrier strips (7) are permanently or releasably fixed to the tampon cover (4) in spaced apart sections (14).

## Revendications

1. Tampon, particulièrement pour l'hygiène féminine, présentant
un noyau absorbant,
un fil de retrait (5) relié au noyau, gui est dirigé dans la direction opposée à la direction d'introduction,
une enveloppe (4) autour du noyau, perméable aux fluides corporels,
l'enveloppe (4) étant pourvue de bandes formant barrières (7 ; 7' ; 7''), de préférence flexibles, pouvant s'écarter de la surface du tampon, et qui présentent chacune un bord libre (9),
caractérisé en ce que les bords libres (9) des bandes formant barrières (7 ; 7' ; 7'') sont dirigées dans la direction d'introduction de sorte que les bandes formant barrières (7 ; 7' ; 7''), lors de l'introduction dans le canal vaginal, s'écartent de la surface du tampon et obstruent un espace libre (11) formé entre la face externe du tampon et la paroi du vagin.

2. Tampon selon la revendication 1, caractérisé en ce que les bandes formant barrières (7, 7') s'étendent autour du tampon (1, 16) dans la direction de la circonférence et: peuvent s'écarter de façon radiale.

3. Tampon selon la revendication 1 ou 2, caractérisé en ce que les bandes formant barrières (7, 7'') sont formées par des pliages (13) du matériau de l'enveloppe (3).

4. Tampon selon la revendication 1 ou 2, caractérisé en ce que les bandes formant barrières (7') sont appliquées sur l'enveloppe (4) par collage, thermosoudage, aiguilletage, estampage ou équivalent.

5. Tampon selon l'une des revendications 1 à 4, caractérisé en ce que les bandes formant barrières (7, 7', 7'') se composent d'un matériau non tissé hydrophobe.

6. Tampon selon l'une des revendications 1 à 4, caractérisé en ce que les bandes formant barrières (7, 7', 7'') se composent d'un matériau non tissé hydrophile.

7. Tampon selon l'une des revendications 1 à 6, caractérisé en ce que les bandes formant barrières (7') sont conçues en deux couches et qu'elles présentent un remplissage [flocons de cellulose (21)] réalisé dans un matériau absorbant.

8. Tampon selon l'une des revendications 1 à 7, caractérisé en ce que les bords libres (9) des bandes formant barrières (7) sont fixés de façon durable ou détachable à l'enveloppe de tampon (4) au niveau de zones espacées entre elles (14).
